# EUROPEAN PATENT APPLICATION

(11) **EP 4 019 091 A1**
(43) Date of publication of application: **29.06.2022**
(21) Application number: 21860798.4
(22) Date of filing: 27.01.2021
(51) Int. Cl.: A61Q 5/12, A61K 8/31, A61K 8/33, A61K 8/34, A61K 8/37

(54) **SOLID HAIR CONDITIONING AGENT COMPOSITION**

(30) Priority: 26.08.2020 JP 2020142399
(71) Applicant: Max Co., Ltd., Yao-shi Osaka 581-0084 (JP)
(72) Inventor: MORI, Norifumi, Yao-shi, Osaka 581-0084 (JP); IMAI, Daisuke, Yao-shi, Osaka 581-0084 (JP); HOSOI, Yoshishige, Yao-shi, Osaka 581-0084 (JP); ONO, Kengo, Yao-shi, Osaka 581-0084 (JP)
(74) Representative: Müller-Boré & Partner Patentanwälte PartG mbB
(86) International application number: PCT/JP2021/002735
(87) International publication number: WO 2022/044366

(57) **Abstract**

An object of the present invention is to provide a solid hair conditioning agent composition that achieves improvement in the application property of an agent. A solid hair conditioning agent composition including cetanol (A1),stearyl alcohol (A2), a cationic surfactant (B), a liquid oil (C), and a nonionic surfactant (D) can improve the application property of an agent.

## Description

### TECHNICAL FIELD

The present invention relates to a solid hair conditioning agent composition. More specifically, the present invention relates to a solid hair conditioning agent composition that achieves an excellent application property of an agent.

### BACKGROUND ART

Hair conditioning agent compositions are generally used for the purpose of improving the feel of hair after cleansing the hair. For this purpose, in a hair conditioning agent composition, a cationic surfactant is widely used that has excellent adsorptivity to hair and has a high effect of imparting softness and/or flexibility to hair. In addition, a higher alcohol and/or an ester oil or the like is generally blended.

For example, Patent Document 1 describes that a hair conditioning agent composition including (a) a cationic surfactant, (b) a higher alcohol, (c) an aromatic acid, (d) a high polymer amino-modified silicone, (e) octyl palmitate or a hydrocarbon oil having a melting point of 35 to 53°C, and (f) water, wherein the molar ratio [(b)/(a)] of the higher alcohol (b) to the cationic surfactant (a) is in the range of 2.5 to 4.0, is excellent in the effect of improving the softness of hair during rinsing.

Patent Document 2 describes that a hair treatment agent composition including (A) an ester including a branched fatty acid and a branched alcohol and having a total carbon number of 30 or less, (B) an amino-polyether-modified silicone, and (C) an ether-based cationic surfactant is excellent in the smoothness during rinsing, and in the finger-combing smoothness and the uniformity of hair during finishing.

Meanwhile, products in which a hair cosmetic is solidified have recently attracted attention from the viewpoint of, for example, environmental problems of plastics. Although most of such products are a solid hair cleanser composition (shampoo bar, solid shampoo), solid hair conditioning agent compositions are also put on the market as shown in Non-Patent Documents 1 and 2, and are designed using cetanol, stearyl alcohol, cocoa butter, a coconut oil, or the like.

### PRIOR ART DOCUMENTS

### NON-PATENT DOCUMENTS

Non-Patent Document 1: ethique Conditioner Bar THE GUARDIAN, [online], P. S. International Co., Ltd., [searched on August 13, 2020], Internet <URL: https://ethicame.com/shop/products/ET000110>
Non-Patent Document 2: ethique little ethique UNTANGLED, [online], P. S. International Co., Ltd., [searched on August 13, 2020], Internet <URL: https://ethicame.com/shop/products/ET001173>

### PATENT DOCUMENTS

Patent Document 1: Japanese Patent Laid-open Publication No. 2014-111574
Patent Document 2: Japanese Patent Laid-open Publication No. 2018-2638

### SUMMARY OF THE INVENTION

### PROBLEMS TO BE SOLVED BY THE INVENTION

A formulation of a hair conditioning agent composition has been designed for the purpose of improving the ability to condition the softness, the smoothness, the uniformity, or the like of hair. Meanwhile, in a formulation of a solid hair conditioning agent composition, molding into a solid is to be assumed, so that the formulation is to be completely different from that of a conventional liquid hair conditioning agent composition. Although currently known formulations of solid hair conditioning agent compositions have succeeded in molding into a solid, sufficient investigation has not been performed with respect to the property to be imparted for the intended use of hair conditioning agent compositions. For example, at the time of use of a solid hair conditioning agent composition, one dose of the agent is to be taken from the solid agent. However, for example, even if the agent is directly applied to hair and put on the hair, a sufficient amount of the agent cannot be taken well as one dose. Thus, ease of use of the composition is much less than that of a liquid hair conditioning agent. Liquid hair conditioning agent compositions essentially do not need the property to achieve adhesion of a sufficient amount of a hair conditioning agent to the surface of a subject when the solid agent is directly applied to the subject (hereinafter, also described as "application property of an agent"), and therefore a technical means to improve such a property is not known.

Therefore, an object of the present invention is to provide a solid hair conditioning agent composition that achieves improvement in the application property of an agent.

### MEANS FOR SOLVING THE PROBLEM

As a result of intensive studies, the present inventor has found that an excellent application property of an agent can be obtained by blending a liquid oil and a nonionic surfactant with a solid hair conditioning agent composition including a predetermined higher alcohol and a cationic surfactant. The present invention has been completed by further studies based on the above-described findings.

Item 1. A solid hair conditioning agent composition including cetanol (A1), stearyl alcohol (A2), a cationic surfactant (B), a liquid oil (C), and a nonionic surfactant (D).

Item 2. The solid hair conditioning agent composition according to the item 1, wherein the liquid oil (C) includes an ester oil (C1) and/or a hydrocarbon oil (C2).

Item 3. The solid hair conditioning agent composition according to the item 2, wherein the ester oil (C1) is a monoester oil (C11).

Item 4. The solid hair conditioning agent composition according to the item 2, wherein the ester oil (C1) includes a triester oil (C13) and/or a tetraester oil (C14).

Item 5. The solid hair conditioning agent composition according to the item 3, wherein the liquid oil (C) includes a triester oil (C13) and/or a tetraester oil (C14) and includes the monoester oil (C11) and/or the hydrocarbon oil (C2).

Item 6. The solid hair conditioning agent composition according to the item 4 or 5, wherein the triester oil (C13) is a castor oil.

Item 7. The solid hair conditioning agent composition according to any one of the items 1 to 6, wherein the nonionic surfactant (D) is selected from the group consisting of polyoxyethylene hydrogenated castor oils (D1), glycerol fatty acid esters (D2), polyoxyalkylene glycerol fatty acid esters (D3), polyalkylene glycol fatty acid esters (D4), sorbitan fatty acid esters (D5), polyoxyalkylene sorbitan fatty acid esters (D6), and polyoxyalkylene alkyl ethers (D7).

Item 8. The solid hair conditioning agent composition according to the item 7, wherein the nonionic surfactant (D) is selected from the group consisting of the polyoxyethylene hydrogenated castor oils (D1), the glycerol fatty acid esters (D2), the polyoxyalkylene glycerol fatty acid esters (D3), and the polyalkylene glycol fatty acid esters (D4).

Item 9. The solid hair conditioning agent composition according to any one of the items 1 to 8, further including behenyl alcohol (A3).

Item 10. The solid hair conditioning agent composition according to any one of the items 1 to 9, wherein the liquid oil (C) is included at a content of 1 to 30% by weight.

Item 11. The solid hair conditioning agent composition according to any one of the items 1 to 10, wherein the nonionic surfactant (D) is included at a content of 1 to 10% by weight.

### ADVANTAGES OF THE INVENTION

According to the present invention, a solid hair conditioning agent composition is provided that achieves improvement in the application property of an agent.

### EMBODIMENTS OF THE INVENTION

The solid hair conditioning agent composition of the present invention is characterized by including cetanol (A1) (hereinafter, also referred to as "component (Al)"), stearyl alcohol (A2) (hereinafter, also referred to as "component (A2)"), a cationic surfactant (B) (hereinafter, also referred to as "component (B)"), a liquid oil (C) (hereinafter, also referred to as "component (C)"), and a nonionic surfactant (D) (hereinafter, also referred to as "component (D)"). Hereinafter, the solid hair conditioning agent composition of the present invention will be described in detail.

### Cetanol (A1) and Stearyl Alcohol (A2)

The solid hair conditioning agent composition of the present invention includes cetanol as the component (A1) and stearyl alcohol as the component (A2).

In the solid hair conditioning agent composition of the present invention, the content of the component (A1) is not particularly limited, and is, for example, 30 to 70% by weight. The content of the component (A1) is preferably 35 to 65% by weight, more preferably 45 to 63% by weight, still more preferably 48 to 61% by weight, even more preferably 50 to 59% by weight, still even more preferably 53 to 58% by weight, and particularly preferably 55 to 58% by weight, from the viewpoint of further improving the application property of an agent, or from, in addition to this viewpoint, the viewpoint of improving the spreadability of the agent and the smoothness of hair during rinsing and/or after drying.

In the solid hair conditioning agent composition of the present invention, the content of the component (A2) is not particularly limited, and is, for example, 5 to 40% by weight. The content of the component (A2) is preferably 10 to 35% by weight, more preferably 15 to 25% by weight, and still more preferably 17 to 20% by weight, from the viewpoint of further improving the application property of an agent, or from, in addition to this viewpoint, the viewpoint of improving the spreadability of the agent and the smoothness of hair during rinsing and/or after drying.

In the solid hair conditioning agent composition of the present invention, the ratio of the component (A1) and that of the component (A2) depend on the contents of the above-described components, and the content of the component (A2) based on 1 part by weight of the component (A1) is, for example, 0.2 to 1 part by weight, preferably 0.24 to 0.5 parts by weight, more preferably 0.26 to 0.45 parts by weight, still more preferably 0.28 to 0.4 parts by weight, and still even more preferably 0.3 to 0.35 parts by weight, from the viewpoint of further improving the application property of an agent, or from, in addition to this viewpoint, the viewpoint of improving the spreadability of the agent and the smoothness of hair during rinsing and/or after drying.

### Behenyl Alcohol (A3)

The solid hair conditioning agent composition of the present invention can further include behenyl alcohol as a component (A3). The behenyl alcohol can be blended for the purpose of improving the smoothness of hair during rinsing.

In a case where the solid hair conditioning agent composition of the present invention includes the component (A3), the content of the component (A3) is not particularly limited, and is, for example, 1 to 30% by weight, preferably 2.5 to 30% by weight, and more preferably 4 to 30% by weight, from the viewpoint of, for example, further improving the smoothness of hair during rinsing. In addition, from the viewpoint of improving the application property of an agent, the spreadability of the agent, and/or the smoothness of the hair after drying, the content is still more preferably 4 to 25% by weight, even more preferably 4 to 10% by weight, and still even more preferably 4 to 8% by weight.

In a case where the solid hair conditioning agent composition of the present invention includes the component (A3), the total amount of the components (A1) to (A3) depends on the contents of the above-described components, and is preferably 60 to 90% by weight, preferably 65 to 86% by weight, more preferably 70 to 83% by weight, and still more preferably 72 to 77% by weight.

In a case where the solid hair conditioning agent composition of the present invention includes the component (A3), the ratio of the total amount of the components (A1) and (A2) and the ratio of the component (A3) depend on the contents of the above-described components, and the content of the component (A3) based on 1 part by weight of the total amount of the components (A1) and (A2) is, for example, 0.02 to 0.55 parts by weight, preferably 0.04 to 0.55 parts by weight, and more preferably 0.06 to 0.55 parts by weight, from the viewpoint of, for example, further improving the smoothness of hair during rinsing. In addition, from the viewpoint of improving the application property of an agent, the spreadability of the agent, and/or the smoothness of the hair after drying, the content is still more preferably 0.06 to 0.5 parts by weight, even more preferably 0.06 to 0.15 parts by weight, and still even more preferably 0.06 to 0.1 parts by weight.

### Cationic Surfactant (B)

The solid hair conditioning agent composition of the present invention includes a cationic surfactant as the component (B).

As the cationic surfactant, components used in hair conditioning compositions can be used without particular limitation. Preferable examples of the cationic surfactant used in the present invention include quaternary ammonium salts represented by the following general formula (I).

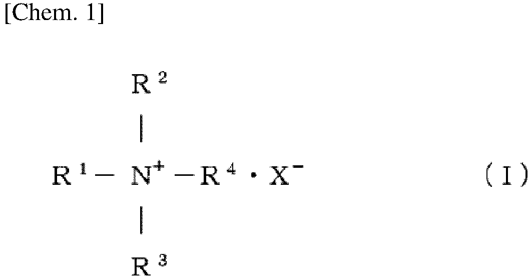

In the formula (I), R¹ represents a linear or branched alkyl group having 6 to 35 carbon atoms and including no ether moiety or an ether moiety. R², R³, and R⁴ each represent a hydrogen atom or a linear or branched alkyl group having 1 to 3 carbon atoms, and may be identical or different. X represents a halogen ion or an organic anion.

Specific examples of R¹ as a linear or branched alkyl group having 6 to 35 carbon atoms and including no ether moiety or an ether moiety include linear or branched alkyl groups having 6 to 35 carbon atoms and including no ether moiety, and linear or branched alkyl groups having 6 to 35 carbon atoms and including an ether moiety. Among the alkyl groups, the linear or branched alkyl groups having 6 to 35 carbon atoms and including no ether moiety are preferable from the viewpoint of further improving the application property of an agent, or from, in addition to this viewpoint, the viewpoint of improving the spreadability of the agent. From the viewpoint of further improving the high-temperature stability, the linear or branched alkyl groups having 6 to 35 carbon atoms and including an ether moiety are preferable.

Specific examples of R¹ in the case of a linear or branched alkyl group having 6 to 35 carbon atoms and including no ether moiety include a hexyl group, a heptyl group, an octyl group, a nonyl group, a decyl group, an undecyl group, a dodecyl group, a tridecyl group, a tetradecyl group, a pentadecyl group, a hexadecyl (cetyl) group, a heptadecyl group, an octadecyl (stearyl) group, a nonadecyl group, an icosyl group, a henicosyl group, a docosyl (behenyl) group, a tricosyl group, a tetracosyl group, a pentacosyl group, a hexacosyl group, a heptacosyl group, an octacosyl group, a nonacosyl group, a triacontyl group, a hentriacontyl group, a dotriacontyl group, a tritriacontyl group, a tetratriacontyl group, a pentatriacontyl group, a myristoleyl group, a palmitoleyl group, an oleyl group, a linoyl group, a linoleyl group, a ricinoleyl group, and an isostearyl group, and alkyl groups having 16 to 22 carbon atoms and including no ether moiety (a hexadecyl (cetyl) group, a heptadecyl group, an octadecyl (stearyl) group, a nonadecyl group, an icosyl group, a henicosyl group, and a docosyl (behenyl) group) are preferable.

Specific examples of R¹ in the case of a linear or branched alkyl group having 6 to 35 carbon atoms and including an ether moiety are to be groups having an ether moiety inserted at an arbitrary position in the above-described specific examples of R¹ in the case of a linear or branched alkyl group including no ether moiety. The number of ether groups in R¹ is not particularly limited, and is, for example, 1 to 5, preferably 1 to 4, more preferably 1 to 3, still more preferably 1 to 2, and still even more preferably 1. More specific examples of R¹ in the case of a linear or branched alkyl group having 6 to 35 carbon atoms and including an ether moiety include alkyl (having 1 to 34, preferably 8 to 28, more preferably 14 to 24, and still more preferably 16 to 20 carbon atoms) oxyalkyl (having 1 to 5 and preferably 2 to 4 carbon atoms) groups.

Specific examples of X in the case of a halogen ion include chlorine, iodine, and bromine, and specific examples of X in the case of an organic anion include methosulfate, ethosulfate, methophosphate, and ethophosphate.

As the component (B), the above-described cationic surfactants may be used singly or in combination of two or more kinds thereof.

More preferable examples of the cationic surfactant used in the present invention include behenyl trimethyl ammonium chloride (= behentrimonium chloride) in which R¹ has 22 carbon atoms, R², R³, and R⁴ are a methyl group, and X is chlorine, stearyltrimethylammonium chloride (= steartrimonium chloride) in which R¹ has 18 carbon atoms, R², R³, and R⁴ are a methyl group, and X is chlorine, cetyltrimethylammonium chloride in which R¹ has 16 carbon atoms, R², R³, and R⁴ are a methyl group, and X is chlorine, and stearoxypropyltrimethylammonium chloride (= stearoxypropyltrimonium chloride) in which R¹ is an alkyl (having 18 carbon atoms) oxyalkyl (having 3 carbon atoms) group, R², R³, and R⁴ are a methyl group, and X is chlorine, and still more preferable examples of the cationic surfactant include behenyl trimethyl ammonium chloride (= behentrimonium chloride) and stearoxypropyltrimethylammonium chloride (= stearoxypropyltrimonium chloride). Behenyl trimethyl ammonium chloride (= behentrimonium chloride) is particularly preferable from the viewpoint of further improving the application property of an agent, or from, in addition to this viewpoint, the viewpoint of improving the spreadability of the agent, and stearoxypropyltrimethylammonium chloride (= stearoxypropyltrimonium chloride) is particularly preferable from the viewpoint of further improving the high-temperature stability.

In the solid hair conditioning agent composition of the present invention, the content of the component (B) is not particularly limited, and is, for example, 1 to 10% by weight. From the viewpoint of further improving the application property of an agent, the content of the component (B) is preferably 2 to 10% by weight, more preferably 4 to 10% by weight, still more preferably 4.5 to 10% by weight, and still even more preferably 5 to 10% by weight. From the viewpoint of further improving the high-temperature stability, the content of the component (B) is preferably 1 to 8% by weight, more preferably 1 to 6% by weight, still more preferably 1 to 5.5% by weight, even more preferably 1 to 5% by weight, still even more preferably 1 to 4.5% by weight, and particularly preferably 1 to 4% by weight. From the viewpoint of achieving both the application property of an agent and the high-temperature stability, the content of the component (B), in a case where R¹ is a linear or branched alkyl group having 6 to 35 carbon atoms and including no ether moiety, is preferably 2 to 8% by weight, more preferably 4 to 6% by weight, still more preferably 4 to 5% by weight, and still even more preferably 4.5 to 5% by weight, and the content, in a case where R¹ is a linear or branched alkyl group having 6 to 35 carbon atoms and including an ether moiety, is preferably 4 to 8% by weight, more preferably 4.5 to 6% by weight, and still more preferably 5 to 5.5% by weight.

### Liquid Oil (C)

The solid hair conditioning agent composition of the present invention includes a liquid oil as the component (C). The liquid oil is an oil that is liquid at 25°C.

Specific examples of the liquid oil include an ester oil (C1) (hereinafter, also described as "component (C1)"), a hydrocarbon oil (C2) (hereinafter, also described as "component (C2)"), a silicone oil (C3), an ether oil (C4), and a fluorine oil (C5). These liquid oils may be used singly or in combination of two or more kinds thereof.

Among the above-described liquid oils, the component (C1) and/or the component (C2) are preferable as the component (C) from the viewpoint of further improving the application property of an agent, or from, in addition to this viewpoint, the viewpoint of improving the spreadability of the agent and the smoothness of hair during rinsing and/or after drying.

Specific examples of the component (C1) as an ester oil include a monoester oil (C11), a diester oil (C12), a triester oil (C13), and a tetraester oil (C14). These ester oils may be used singly or in combination of two or more kinds thereof.

The monoester oil as the component (C11) is not particularly limited, and examples of the monoester oil include monoesters of an aliphatic monocarboxylic or dicarboxylic acid having 2 to 24 carbon atoms, and monoesters of an aromatic monocarboxylic or dicarboxylic acid. Specific examples of the monoester oil include cetyl 2-ethylhexanoate, cetyl octanoate, isononyl isononanoate, isotridecyl isononanoate, hexyl laurate, isopropyl myristate, octyldodecyl myristate, myristyl myristate, 2-hexyldecyl myristate, isopropyl palmitate, octyl palmitate, 2-hexyldecyl palmitate, butyl stearate, isocetyl stearate, isocetyl isostearate, decyl oleate, isodecyl benzoate, octyl methoxycinnamate, hexyldecyl dimethyloctanoate, cetyl lactate, myristyl lactate, acetylated lanolin, 2-ethylhexyl succinate, 2-hexyldecyl adipate, and C12-15 alkyl benzoate. These monoester oils may be used singly or in combination of two or more kinds thereof. Among these monoester oils, the esters of an aliphatic monocarboxylic acid having 2 to 24 carbon atoms are preferable, and cetyl 2-ethylhexanoate is more preferable, from the viewpoint of further improving the application property of an agent, or from, in addition to this viewpoint, the viewpoint of improving the spreadability of the agent and the smoothness of hair during rinsing and/or after drying.

The diester oil as the component (C12) is not particularly limited, and examples of the diester oil include diesters of a dicarboxylic acid having 3 to 18 carbon atoms and di-fatty acid esters of a polyhydric alcohol, and specific examples of the diester oil include propylene glycol dicaprylate, neopentyl glycol dicaprate, glycol distearate, propylene glycol diisostearate, glyceryl diisostearate, glyceryl monoisostearate monomyristate, glyceryl di-2-heptylundecanoate, di-2-ethylhexyl succinate, diisopropyl sebacate, diisostearyl malate, ethylene glycol di-2-ethylhexanoate, diisobutyl adipate, di-2-heptylundecyl adipate, and di-2-ethylhexyl sebacate. These diester oils may be used singly or in combination of two or more kinds thereof.

The triester oil as the component (C13) is a tri-fatty acid ester of a trihydric or higher polyhydric alcohol, and may be a synthetic oil or a natural oil. Specific examples of the triester oil include glyceryl trimyristate, glyceryl triisopalmitate, glyceryl tri-2-heptylundecanoate, trimethylolpropane triethylhexanoate, trimethylolpropane trioctanoate, glyceryl tri(caprylate/caprate), glyceryl trioleate, glyceryl tri-2-ethylhexanoate, glyceryl triisostearate, and vegetable oils (such as a camellia oil, a sesame oil, a castor oil, an olive oil, and a jojoba oil). These triester oils may be used singly or in combination of two or more kinds thereof. Among these triester oils, the tri-fatty acid esters of a trihydric alcohol (triglycerides) are preferable, vegetable oils are more preferable, and a castor oil is still more preferable, from the viewpoint of further improving the application property of an agent, or from, in addition to this viewpoint, the viewpoint of improving the spreadability of the agent and the smoothness of hair during rinsing and/or after drying.

Examples of the tetraester oil as the component (C14) include tetra-fatty acid esters of a tetrahydric or higher polyhydric alcohol, and specific examples of the tetraester oil include pentaerythrityl tetra(behenate/benzoate/ethylhexanoate), pentaerythrityl tetraethylhexanoate, pentaerythrityl tetraoctanoate, and pentaerythritol tetra-2-ethylhexanoate. These tetraester oils may be used singly or in combination of two or more kinds thereof.

Among the components (C1), the component (C11) is preferable, the esters of an aliphatic monocarboxylic acid having 2 to 24 carbon atoms are more preferable, and cetyl 2-ethylhexanoate is more preferable, particularly from the viewpoint of further improving the application property of an agent, or from, in addition to this viewpoint, the viewpoint of improving the spreadability of the agent and/or the smoothness of hair during rinsing.

Specific examples of the component (C2) as a hydrocarbon oil include linear or branched hydrocarbon oils. More specific examples of the component (C2) include liquid paraffin, liquid isoparaffin, squalane, and squalene, and liquid paraffin is preferable from the viewpoint of further improving the application property of an agent, or from, in addition to this viewpoint, the viewpoint of improving the spreadability of the agent and/or the smoothness of hair during rinsing.

Examples of the silicone oil as the component (C3) include dimethylpolysiloxane, cyclomethicone, dimethicone, trisiloxane methyltrimethicone, ethyltrisiloxane, dimethylcyclopolysiloxane, methylphenylpolysiloxane, methylhydrogenpolysiloxane, and higher alcohol-modified organopolysiloxane. Examples of the ether oil as the component (C4) include alkyl-1,3-dimethylbutyl ethers, dicaprylyl ether, and dicaprylyl ether. Examples of the fluorine oil as the component (C5) include fluoropolyethers and perfluoroalkyl ether silicone.

Among the components (C), the components (C13) and (C14) having many ester chains in the molecule are preferable, and as the component (C13), vegetable oils are more preferable, and a castor oil is still more preferable, from the viewpoint of obtaining excellent high-temperature stability even in a case where the component (C) is blended in a relatively large amount (of, for example, 9% by weight or more, 10% by weight or more, 15% by weight or more, or 20% by weight or more) in the solid hair conditioning agent composition.

In the solid hair conditioning agent composition of the present invention, the component (C) is more preferably obtained by combining the component (C13) and/or the component (C14) with the component (C11) and/or the component (C2), particularly preferably obtained by combining the component (C13) with the component (C11) and/or the component (C2), and most preferably obtained by combining a castor oil with the component (C11) and/or the component (C2), from the viewpoint of the application property of an agent, or from the viewpoint of achieving, in addition to the application property, excellence in the spreadability of the agent, the smoothness of hair during rinsing and/or after drying, and the high-temperature stability in a good balance.

In the solid hair conditioning agent composition of the present invention, the content of the component (C) is not particularly limited, and is to be determined according to the degree of the application property of an agent to be obtained, or according to, in addition to the degree of the application property, the degree of spreadability of the agent, and the degree of smoothness of hair during rinsing and/or after drying. The content of the component (C) is, for example, 1 to 30% by weight. The content of the component (C) is preferably 3 to 30% by weight, more preferably 5 to 30% by weight, still more preferably 8 to 30% by weight, even more preferably 10 to 30% by weight, still even more preferably 15 to 30% by weight, and particularly preferably 20 to 30% by weight, from the viewpoint of further improving the application property of an agent, or from, in addition to this viewpoint, the viewpoint of improving the spreadability of the agent and the smoothness of hair during rinsing and/or after drying. From the viewpoint of improving the high-temperature stability, the content of the component (C) is preferably 1 to 20% by weight, more preferably 1 to 15% by weight, still more preferably 1 to 13.5% by weight, and still even more preferably 1 to 10% by weight.

In the solid hair conditioning agent composition of the present invention, the ratio of the components (A1) and (A2) and that of the component (C) depend on the contents of the above-described components, and the content of the component (C) based on 1 part by weight of the total amount of the components (A1) and (A2) is, for example, 0.02 to 0.35 parts by weight. The content of the component (C) based on 1 part by weight of the total amount of the component (A2) is preferably 0.04 to 0.35 parts by weight, more preferably 0.06 to 0.35 parts by weight, still more preferably 0.1 to 0.35 parts by weight, even more preferably 0.13 to 0.35 parts by weight, still even more preferably 0.15 to 0.31 parts by weight, and particularly preferably 0.17 to 0.35 parts by weight, from the viewpoint of further improving the application property of an agent, or from, in addition to this viewpoint, the viewpoint of improving the spreadability of the agent and the smoothness of hair during rinsing and/or after drying. From the viewpoint of improving the high-temperature stability, the content of the component (C) is preferably 0.02 to 0.3 parts by weight, more preferably 0.02 to 0.22 parts by weight, and still more preferably 0.02 to 0.18 parts by weight.

In the case of combining the component (C13) and/or the component (C14) with the component (C11) and/or the component (C2), the ratio of the component (C13) and/or the component (C14) and that of the total amount of the components (C11) and (C2) are not particularly limited, and the total amount of the components (C11) and (C2) based on 1 part by weight of the total amount of the components (C13) and (C14) is, for example, 0.05 to 1.2 parts by weight. The total amount of the component (C11) and/or the component (C2) based on 1 part by weight of the total amount of the components (C13) and (C14) is preferably 0.05 to 1 part by weight, preferably 0.05 to 0.8 parts by weight, more preferably 0.05 to 0.6 parts by weight, and still more preferably 0.05 to 0.4 parts by weight, from the viewpoint of further improving the application property of an agent, or from, in addition to this viewpoint, the viewpoint of improving the smoothness of hair after drying. From the viewpoint of improving the high-temperature stability, the total amount of the component (C11) and/or the component (C2) based on 1 part by weight of the total amount of the components (C13) and (C14) is more preferably 0.1 to 0.4 parts by weight, still more preferably 0.18 to 0.4 parts by weight, and particularly preferably 0.2 to 0.4 parts by weight or 0.2 to 0.35 parts by weight.

### Nonionic Surfactant (D)

The solid hair conditioning agent composition of the present invention includes a nonionic surfactant as the component (D).

Specific examples of the nonionic surfactant include polyoxyethylene hydrogenated castor oils (D1), glycerol fatty acid esters (D2), polyoxyalkylene glycerol fatty acid esters (D3), polyalkylene glycol fatty acid esters (D4), sorbitan fatty acid esters (D5), polyoxyalkylene sorbitan fatty acid esters (D6), and polyoxyalkylene alkyl ethers (D7). These nonionic surfactants may be used singly or in combination of two or more kinds thereof.

The number of moles of ethylene oxide added to the polyoxyethylene hydrogenated castor oil as the component (D1) is not particularly limited, and is, for example, 5 to 100. The number of moles of ethylene oxide added to the polyoxyethylene hydrogenated castor oil is preferably 10 to 80, more preferably 20 to 70, still more preferably 30 to 65, still even more preferably 40 to 60, and particularly preferably 45 to 55, from the viewpoint of further improving the application property of an agent, or from, in addition to this viewpoint, the viewpoint of improving the spreadability of the agent and the smoothness of hair during rinsing and/or after drying. These polyoxyethylene hydrogenated castor oils may be used singly or in combination of two or more kinds thereof.

The glycerol fatty acid esters as the component (D2) include monoglycerol fatty acid esters and polyglycerol fatty acid esters. These glycerol fatty acid esters may be used singly or in combination of two or more kinds thereof. Among these glycerol fatty acid esters, polyglycerol fatty acid esters are preferable from the viewpoint of further improving the application property of an agent, or from, in addition to this viewpoint, the viewpoint of improving the spreadability of the agent and the smoothness of hair during rinsing and/or after drying.

The monoglycerol fatty acid esters are not particularly limited, and examples thereof include esters of a fatty acid having 8 to 22 carbon atoms and glycerol, and specific examples thereof include glyceryl monocaprylate, glyceryl monocaprate, glyceryl monolaurate, glyceryl monomyristate, glyceryl monopalmitate, glyceryl monostearate, glyceryl monoisostearate, glyceryl monobehenate, glyceryl monooleate, glyceryl monoerucate, glyceryl sesquioleate, glyceryl distearate, glyceryl diisostearate, and glyceryl diarachidate. These monoglycerol fatty acid esters may be used singly or in combination of two or more kinds thereof.

The polyglycerol fatty acid esters are not particularly limited, and examples thereof include esters of a fatty acid having 8 to 22 carbon atoms and a polyglycerol having a polymerization degree of 2 to 10, preferably 4 to 10, more preferably 6 to 10, still more preferably 8 to 10, and particularly preferably 9 to 10, and specific examples thereof include diglyceryl monocaprylate, decaglyceryl monocaprylate, hexaglyceryl monocaprate, tetraglyceryl monolaurate, hexaglyceryl monolaurate, decaglyceryl monolaurate, polyglyceryl (polymerization degree: 4 to 10, preferably 6 to 10, more preferably 8 to 10, and still more preferably 9 to 10) monolaurate, decaglyceryl monomyristate, decaglyceryl monostearate, decaglyceryl monoisostearate, polyglyceryl (polymerization degree: 2 to 10) monostearate, diglyceryl monooleate, hexaglyceryl monooleate, diglyceryl sesquioleate, polyglyceryl (polymerization degree: 2 to 10) diisostearate, polyglyceryl (polymerization degree: 6 to 10) distearate, diglyceryl triisostearate, and polyglyceryl (polymerization degree: 10) tristearate. These polyglycerol fatty acid esters may be used singly or in combination of two or more kinds thereof.

The polyoxyalkylene glycerol fatty acid ester as the component (D3) is not particularly limited, and examples of the polyoxyalkylene glycerol fatty acid ester include esters of a glycerol fatty acid ester described above as the component (D2) and a polyalkylene glycol. The number of moles of alkylene oxide added in the polyoxyalkylene glycerol fatty acid ester is, for example, 5 to 20, preferably 10 to 18, and more preferably 13 to 16. Preferable examples of the polyoxyalkylene glycerol fatty acid ester include polyoxyethylene glyceryl monostearate and polyoxyethylene glyceryl monooleate. These polyoxyalkylene glycerol fatty acid esters may be used singly or in combination of two or more kinds thereof.

The polyalkylene glycol fatty acid ester as the component (D4) is not particularly limited, and examples of the polyalkylene glycol fatty acid ester include esters of a polyalkylene glycol and a fatty acid having 8 to 22 carbon atoms. The number of moles of alkylene oxide added in the polyalkylene glycol fatty acid ester is, for example, 2 to 20, preferably 4 to 18, more preferably 6 to 16, still more preferably 8 to 14, and still even more preferably 9 to 12. Specific examples of the polyalkylene glycol fatty acid ester include polyethylene glycol fatty acid esters and propylene glycol fatty acid esters. Specific examples of the polyethylene glycol fatty acid esters include polyethylene glycol monooleate, polyethylene glycol monostearate, and polyethylene glycol monolaurate. Specific examples of the propylene glycol fatty acid esters include propylene glycol monostearate, propylene glycol monolaurate, and propylene glycol monooleate. These polyalkylene glycol fatty acid esters may be used singly or in combination of two or more kinds thereof.

The sorbitan fatty acid ester as the component (D5) is not particularly limited, and examples of the sorbitan fatty acid ester include esters of sorbitan and a fatty acid having 8 to 22 carbon atoms, and specific examples of the sorbitan fatty acid ester include sorbitan monooleate, sorbitan monostearate, sorbitan monoisostearate, sorbitan monopalmitate, sorbitan monolaurate, sorbitan trioleate, sorbitan tristearate, sorbitan sesquistearate, sorbitan sesquioleate, sorbitan sesquiisostearate, and sorbitan cocoate. These sorbitan fatty acid esters may be used singly or in combination of two or more kinds thereof.

The polyoxyalkylene sorbitan fatty acid ester as the component (D6) is not particularly limited, and examples of the polyoxyalkylene sorbitan fatty acid ester include esters of a sorbitan fatty acid ester described above as the component (D5) and a polyalkylene glycol. The number of moles of alkylene oxide added in the polyoxyalkylene sorbitan fatty acid ester is, for example, 10 to 30, preferably 15 to 25, and more preferably 18 to 22. Preferable examples of the polyoxyalkylene sorbitan fatty acid ester include polyoxyethylene sorbitan monolaurate, polyoxyethylene sorbitan monopalmitate, polyoxyethylene sorbitan monostearate, polyoxyethylene sorbitan monooleate, polyoxyethylene sorbitan monoisostearate, polyoxyethylene sorbitan monococoate, polyoxyethylene sorbitan tristearate, and polyoxyethylene sorbitan trioleate. More preferable examples of the polyoxyalkylene sorbitan fatty acid ester include polysorbate 20 (polyoxyethylene (20) sorbitan monolaurate), polysorbate 60 (polyoxyethylene (20) sorbitan monostearate), polysorbate 65 (polyoxyethylene (20) sorbitan tristearate), and polysorbate 80 (polyoxyethylene (20) sorbitan monooleate). These polyoxyalkylene sorbitan fatty acid esters may be used singly or in combination of two or more kinds thereof.

The polyoxyalkylene alkyl ether as the component (D7) is not particularly limited, and examples of the polyoxyalkylene alkyl ether include ethers of a polyalkylene glycol and an alcohol having 8 to 22 carbon atoms. The number of moles of alkylene oxide added in the polyoxyalkylene alkyl ether is, for example, 5 to 40, preferably 5 to 30, more preferably 10 to 25, still more preferably 15 to 22, and still even more preferably 18 to 22. Specific examples of the polyoxyalkylene alkyl ether include polyoxyethylene alkyl ethers and polyoxypropylene alkyl ethers. Preferable examples of the polyoxyalkylene alkyl ether include polyoxyethylene lauryl ether, polyoxyethylene cetyl ether, polyoxyethylene oleyl cetyl ether, polyoxyethylene stearyl ether, polyoxyethylene oleyl ether, polyoxyethylene polyoxypropylene lauryl ether, polyoxypropylene cetyl ether, polyoxypropylene isocetyl ether, polyoxypropylene stearyl ether, and polyoxypropylene oleyl ether. More preferable examples of the polyoxyalkylene alkyl ether include ceteth-20 (polyoxyethylene (20) cetyl ether) and cetoleth-20 (polyoxyethylene (20) oleyl cetyl ether). These polyoxyalkylene alkyl ethers may be used singly or in combination of two or more kinds thereof.

Among these components (D), the polyoxyethylene hydrogenated castor oils (D1), the glycerol fatty acid esters (D2), the polyoxyalkylene glycerol fatty acid esters (D3), and the polyalkylene glycol fatty acid esters (D4) are preferable from the viewpoint of further improving the application property of an agent.

In the solid hair conditioning agent composition of the present invention, the content of the component (D) is not particularly limited, and is to be determined according to the degree of the application property of an agent to be obtained, or according to, in addition to the degree of the application property, the degree of spreadability of the agent, and the degree of smoothness of hair during rinsing and/or after drying. The content of the component (D) is, for example, 1 to 10% by weight. The content of the component (D) is preferably 2 to 10% by weight, and more preferably 3 to 10% by weight, 3.5 to 8% by weight, 4 to 6% by weight, or 4.5 to 5.5% by weight from the viewpoint of further improving the application property of an agent, or from, in addition to this viewpoint, the viewpoint of improving the spreadability of the agent and the smoothness of hair during rinsing and/or after drying.

In the solid hair conditioning agent composition of the present invention, the ratio of the components (A1) and (A2) and that of the component (D) depend on the contents of the above-described components, and the content of the component (D) based on 1 part by weight of the total amount of the components (A1) and (A2) is, for example, 0.01 to 0.15 parts by weight. The content of the component (D) is preferably 0.03 to 0.15 parts by weight, more preferably 0.035 to 0.15 parts by weight, and still more preferably 0.04 to 0.15 parts by weight, 0.053 to 0.12 parts by weight, or 0.06 to 0.072 parts by weight from the viewpoint of further improving the application property of an agent, or from, in addition to this viewpoint, the viewpoint of improving the spreadability of the agent and the smoothness of hair during rinsing and/or after drying.

### Another Component

As long as an effect of the present invention is not impaired, the solid hair conditioning agent composition of the present invention may include an additive component usually used in the technical field of hair conditioning agent compositions in addition to the above-described components (A1),(A2), (B), (C), and (D), and the component (A3) that is blended if necessary. Examples of such an additive component include higher alcohols (alcohols having 12 or more carbon atoms, other than the components (A1) to (A3)), medium alcohols (alcohols having 6 to 11 carbon atoms), lower alcohols (having 1 to 5 carbon atoms), polyhydric alcohols, solid fats, amphoteric surfactants, thickeners, hair quality improvers, aroma chemicals, antiseptics, ultraviolet absorbers, antioxidants, and bactericides.

In a case where the solid hair conditioning agent composition of the present invention includes a higher alcohol other than the components (A1) to (A3), the total amount of the components (A1) to (A3) and the higher alcohol is preferably 60 to 90% by weight, preferably 65 to 86% by weight, more preferably 70 to 83% by weight, and still more preferably 75 to 80% by weight.

The solid hair conditioning agent composition of the present invention preferably includes no water although a case is not excluded in which the solid hair conditioning agent composition includes water. The solid hair conditioning agent composition including substantially no water includes no water or includes entrained moisture in a minute amount (of, for example, 0.01% by weight or less and preferably 0.001% by weight or less), and preferably includes no water.

### Intended Use

The solid hair conditioning agent composition of the present invention is used for treatment of the surface of hair. Specifically, the solid hair conditioning agent composition of the present invention can be used as a hair cosmetic to be washed off, such as a hair rinse, a hair pack, a hair conditioner, or a hair treatment agent. In use of the solid hair conditioning agent composition, an appropriate amount of the agent from the solid hair conditioning agent composition can be adhered and applied to hair. Examples of the method of taking an appropriate amount of the agent include a method in which the solid hair conditioning agent composition is rubbed against a palm to take the agent, and a method in which the solid hair conditioning agent composition is directly applied to hair.

### Production Method

To produce the solid hair conditioning agent composition of the present invention, the components (A1), (A2), (B), (C), and (D), the component (A3) that is blended if necessary, and another component that is blended if necessary are heated, melted, and mixed, and the resulting mixture is cooled and solidified.

### Examples

Hereinafter, the present invention will be described more specifically with reference to Examples, but the present invention is not limited thereto.

Solid hair conditioning agent compositions (50 g/piece, 5 cm × 5 cm × 2.5 cm) shown in Tables 1 to 4 were prepared. Specifically, the components shown in Tables 1 to 4 were melted and mixed at 75°C, the resulting mixture was poured into a mold, naturally cooled to room temperature, and then removed from the mold to produce a solid hair conditioning agent composition.

With respect to the obtained solid hair conditioning agent composition, the application property of the agent to hair, the spreadability of the agent on the hair, the smoothness during rinsing, and the smoothness of the hair after drying were evaluated as follows. Tables 1 to 4 show the results.

### <Application Property of Agent to Hair>

The solid hair conditioning agent composition was applied to hair wetted with water, in one direction from the root toward the tip of the hair 6 times, and the amount of the agent put on the hair surface was evaluated on a scale of 1 to 10 so that 10 points were given (the amount of the agent put on the hair was evaluated to be sufficient and the solid hair conditioning agent composition was evaluated to be very easy to use) in the case of the solid hair cleanser composition in Example 22, and 1 point was given in a case where no agent was put on hair. The greater the score is, the more excellent the application property of the agent to hair is determined to be. In a case where the score was 4 points or more, the application property of the agent to hair was determined to be improved to a desired degree.

### <Spreadability of Agent on Hair>

The solid hair conditioning agent composition was applied to the surface of hair wetted with water until a sufficient amount of the agent was put on the hair (the solid hair conditioning agent composition given a score of 1 to 3 points in the application property test was repeatedly applied until the amount was such that the score of the application property was 4 points), and the agent put on the hair surface was spread by hand. The degree of spreadability of the agent on hair was evaluated on a scale 1 to 10 so that 10 points were given in the case of the solid hair cleanser composition in Example 22, and 1 point was given in a case where the agent was not spread. The greater the score is, the more excellent the spreadability of the agent on hair is determined to be.

### <Smoothness during Rinsing>

Previously, 1.0 g of the solid hair conditioning agent composition was spread with an appropriate amount of water. The spread solid hair conditioning agent composition was adhered to hair, and rinsed with hot water (38°C). The smoothness of hair during rinsing was evaluated on a scale 1 to 10 so that 10 points were given (the hair was evaluated to be very smooth) in the case of the solid hair cleanser composition in Example 22, and 1 point was given (the hair was evaluated to be rough) in the case of the solid hair conditioning agent composition in Comparative Example 5. When the score is greater, the solid hair conditioning agent composition is determined to provide more excellent smoothness during rinsing.

### <Smoothness of Hair after Drying>

After the above-described test for the smoothness during rinsing, the hair was dried, and the smoothness of the hair after drying was evaluated on a scale 1 to 10 so that 10 points were given (the hair was evaluated to be very smooth) in the case of the solid hair cleanser composition in Example 22, and 1 point was given in a case where the hair was rough. When the score is greater, the solid hair conditioning agent composition is determined to provide more excellent smoothness of hair after drying.

### <High-Temperature Stability>

The solid hair conditioning agent composition was left to stand for 72 hours under storage conditions of a temperature of 40°C and a humidity of 75% RH. Then, the surface condition (presence or absence of stickiness and the degree of stickiness) of the solid hair conditioning agent composition was evaluated on a scale 1 to 10. Specifically, 10 points were given in a case where the surface was not sticky at all, 1 point was given in a case where the stickiness was so large that the solid agent lost shape retention (the solid agent itself was melted), 2 points were given in a case where the solid agent kept shape retention although the stickiness was extremely large, 9 points were given in a case where extremely slight stickiness was only observed, and the degree of stickiness was evaluated into 8 levels on a scale 2 to 9. When the score is greater, the solid hair conditioning agent composition is determined to provide more excellent high-temperature stability.

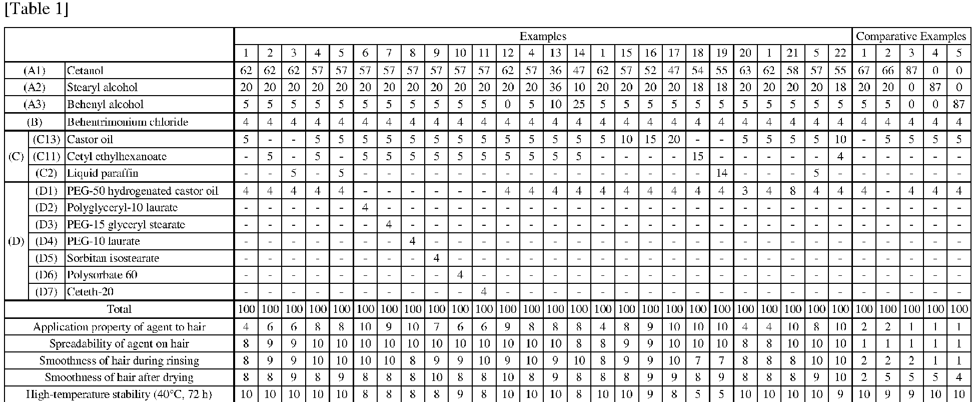

Table shows value of blending amount of each component by unit of % by weight.

**[Table 2]**

| | | | Examples | | | |
|---|---|---|---|---|---|---|
| | | | 23 | 5 | 24 | 25 |
| (A1) | | Cetanol | 58 | 57 | 56.5 | 56 |
| (A2) | | Stearyl alcohol | 20 | 20 | 20 | 20 |
| (A3) | | Behenyl alcohol | 5 | 5 | 5 | 5 |
| (B) | | Behentrimonium chloride | 3 | 4 | 4.5 | 5 |
| (C) | (C13) | Castor oil | 5 | 5 | 5 | 5 |
| | (C11) | Cetyl ethylhexanoate | - | - | - | - |
| | (C2) | Liquid paraffin | 5 | 5 | 5 | 5 |
| (D) | (D1) | PEG-50 hydrogenated castor oil | 4 | 4 | 4 | 4 |
| | (D2) | Polyglyceryl-10 laurate | - | - | - | - |
| | (D3) | PEG-15 glyceryl stearate | - | - | - | - |
| | (D4) | PEG-10 laurate | - | - | - | - |
| | (D5) | Sorbitan isostearate | - | - | - | - |
| | (D6) | Polysorbate 60 | - | - | - | - |
| | (D7) | Ceteth-20 | - | - | - | - |
| Total | | | 100 | 100 | 100 | 100 |
| Application property of agent to hair | | | 7 | 8 | 9 | 9 |
| Spreadability of agent on hair | | | 10 | 10 | 10 | 10 |
| Smoothness of hair during rinsing | | | 10 | 10 | 10 | 10 |
| Smoothness of hair after drying | | | 9 | 9 | 9 | 9 |
| High-temperature stability (40°C, 72 h) | | | 10 | 10 | 9 | 8 |

Table shows value of blending amount of each component by unit of % by weight.

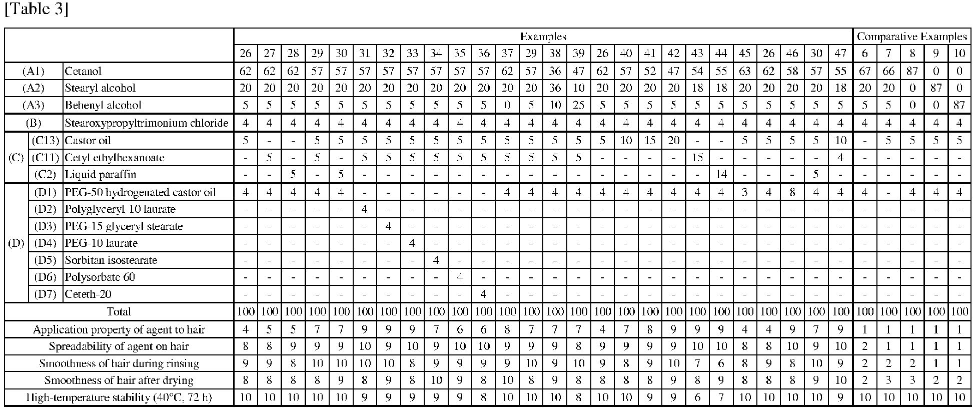

Table shows value of blending amount of each component by unit of % by weight.

**[Table 4]**

| | | | Examples | | | |
|---|---|---|---|---|---|---|
| | | | 30 | 48 | 49 | 50 |
| (A1) | | Cetanol | 57 | 56 | 55.5 | 55 |
| (A2) | | Stearyl alcohol | 20 | 20 | 20 | 20 |
| (A3) | | Behenyl alcohol | 5 | 5 | 5 | 5 |
| (B) | | Stearoxypropyltrimonium chloride | 4 | 5 | 5.5 | 6 |
| (C) | (C13) | Castor oil | 5 | 5 | 5 | 5 |
| | (C11) | Cetyl ethylhexanoate | - | - | - | - |
| | (C2) | Liquid paraffin | 5 | 5 | 5 | 5 |
| (D) | (D1) | PEG-50 hydrogenated castor oil | 4 | 4 | 4 | 4 |
| | (D2) | Polyglyceryl-10 laurate | - | - | - | - |
| | (D3) | PEG-15 glyceryl stearate | - | - | - | - |
| | (D4) | PEG-10 laurate | - | - | - | - |
| | (D5) | Sorbitan isostearate | - | - | - | - |
| | (D6) | Polysorbate 60 | - | - | - | - |
| | (D7) | Ceteth-20 | - | - | - | - |
| Total | | | 100 | 100 | 100 | 100 |
| Application property of agent to hair | | | 7 | 8 | 9 | 9 |
| Spreadability of agent on hair | | | 9 | 10 | 10 | 10 |
| Smoothness of hair during rinsing | | | 10 | 10 | 10 | 10 |
| Smoothness of hair after drying | | | 9 | 9 | 9 | 9 |
| High-temperature stability (40°C, 72 h) | | | 10 | 10 | 10 | 8 |

Table shows value of blending amount of each component by unit of % by weight.

## Claims

1. A solid hair conditioning agent composition comprising:
cetanol (A1);
stearyl alcohol (A2);
a cationic surfactant (B);
a liquid oil (C); and
a nonionic surfactant (D).

2. The solid hair conditioning agent composition according to claim 1, wherein the liquid oil (C) includes an ester oil (C1) and/or a hydrocarbon oil (C2).

3. The solid hair conditioning agent composition according to claim 2, wherein the ester oil (C1) is a monoester oil (C11).

4. The solid hair conditioning agent composition according to claim 2, wherein the ester oil (C1) includes a triester oil (C13) and/or a tetraester oil (C14).

5. The solid hair conditioning agent composition according to claim 3, wherein the liquid oil (C) includes a triester oil (C13) and/or a tetraester oil (C14) and includes the monoester oil (C11) and/or the hydrocarbon oil (C2).

6. The solid hair conditioning agent composition according to claim 4 or 5, wherein the triester oil (C13) is a castor oil.

7. The solid hair conditioning agent composition according to any one of claims 1 to 6, wherein the nonionic surfactant (D) is selected from the group consisting of polyoxyethylene hydrogenated castor oils (D1), glycerol fatty acid esters (D2), polyoxyalkylene glycerol fatty acid esters (D3), polyalkylene glycol fatty acid esters (D4), sorbitan fatty acid esters (D5), polyoxyalkylene sorbitan fatty acid esters (D6), and polyoxyalkylene alkyl ethers (D7).

8. The solid hair conditioning agent composition according to claim 7, wherein the nonionic surfactant (D) is selected from the group consisting of the polyoxyethylene hydrogenated castor oils (D1), the glycerol fatty acid esters (D2), the polyoxyalkylene glycerol fatty acid esters (D3), and the polyalkylene glycol fatty acid esters (D4).

9. The solid hair conditioning agent composition according to any one of claims 1 to 8, further comprising behenyl alcohol (A3).

10. The solid hair conditioning agent composition according to any one of claims 1 to 9, wherein the liquid oil (C) is included at a content of 1 to 30% by weight.

11. The solid hair conditioning agent composition according to any one of claims 1 to 10, wherein the nonionic surfactant (D) is included at a content of 1 to 10% by weight.
